(19) Europäisches Patentamt
European Patent Office
Office européen des brevets

(11) EP 1 244 663 B1

(12) **EUROPEAN PATENT SPECIFICATION**

(45) Date of publication and mention of the grant of the patent:
**25.08.2004 Bulletin 2004/35**

(21) Application number: **00989116.9**

(22) Date of filing: **19.12.2000**

(51) Int Cl.[7]: **C07D 471/04**, A61K 31/5025

(86) International application number:
**PCT/SE2000/002606**

(87) International publication number:
**WO 2001/047923 (05.07.2001 Gazette 2001/27)**

(54) **COMPOUNDS AND METHODS FOR THE TREATMENT OF PAIN**
VERBINDUNGEN UND VERFAHREN ZUR BEHANDLUNG VON SCHMERZ
COMPOSES ET PROCEDES POUR LE TRAITEMENT DE LA DOULEUR

(84) Designated Contracting States:
**AT BE CH CY DE DK ES FI FR GB GR IE IT LI LU MC NL PT SE TR**

(30) Priority: **23.12.1999 US 171906 P**
**29.09.2000 US 236880 P**

(43) Date of publication of application:
**02.10.2002 Bulletin 2002/40**

(73) Proprietor: **AstraZeneca AB**
**151 85 Södertälje (SE)**

(72) Inventors:
- **BROWN, Dean Gordon, AstraZeneca Wilmington, DE 19850-5437 (US)**
- **XIAO, Wenhua, AstraZeneca Wilmington, DE 19850-5437 (US)**
- **URBANEK, Rebecca Ann, AstraZeneca Wilmington, DE 19850-5437 (US)**
- **MURPHY, Megan, AstraZeneca Wilmington, DE 19850-5437 (US)**
- **BARE, Thomas, Michael West Chester, PA 19382 (US)**

(56) References cited:
**EP-A1- 0 736 531**
**WO-A1-95/11244**
**WO-A1-96/15127**

- **BARE T.M.: 'PYRIDAZINO[4,5-B]QUINOLINEDIONES: NOVEL GLYCINE/N-METHYLD-ASPARTATE ANTAGONISTS FOR THE TREATMENT OF STROKE' JOURNAL OF HETEROCYCLIC CHEMISTRY vol. 35, September 1998, pages 1171 - 1186**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 244 663 B1

## Description

### Field of the Invention

This invention relates to the treatment or prevention of pain or nociception.

### Related Art

Pain is a sensory experience distinct from sensations of touch, pressure, heat and cold. It is often described by sufferers by such terms as bright, dull, aching, pricking, cutting or burning and is generally considered to include both the original sensation and the reaction to that sensation. This range of sensations, as well as the variation in perception of pain by different individuals, renders a precise definition of pain difficult, however, many individuals suffer with severe and continuous pain.

Pain that is caused by damage to neural structures is often manifest as a neural supersensitivity or hyperalgesia and is termed "neuropathic" pain. Pain can also be "caused" by the stimulation of nociceptive receptors and transmitted over intact neural pathways, such pain is termed "nociceptive" pain.

The level of stimulation at which pain becomes noted is referred to as the "pain threshold." Analgesics are pharmaceutical agents which relieve pain by raising the pain threshold without a loss of consciousness. After administration of an analgesic drug a stimulus of greater intensity or longer duration is required before pain is experienced. In an individual suffering from hyperalgesia an analgesic drug may have an anti-hyperalgesic effect. In contrast to analgesics, agents such as local anaesthetics block transmission in peripheral nerve fibers thereby blocking awareness of pain. General anaesthetics, on the other hand, reduce the awareness of pain by producing a loss of consciousness.

Tachykinin antagonists have been reported to induce antinociception in animals, which is believed to be analogous to analgesia in man (Maggi et al, J. Auton. Pharmacol. (1993) 13, 23-93). In particular, non-peptide NK-1 receptor antagonists have been shown to produce such analgesia. For example, the NK-1 receptor antagonist RP 67,580 produced analgesia with potency comparable to that of morphine (Garret et al, Proc. Natl. Acad. Sci. USA (1993) 88, 10208-10212).

The opioid analgesics are a well-established class of analgesic agents with morphine-like actions. Synthetic and semi-synthetic opioid analgesics are derivatives of five chemical classes of compound: phenanthrenes; phenylheptylamines; phenylpiperidines; morphinans; and benzomorphans. Pharmacologically these compounds have diverse activities, thus some are strong agonists at the opioid receptors (e.g. morphine); others are moderate to mild agonists (e.g. codeine); still others exhibit mixed agonist-antagonist activity (e.g. nalbuphine); and yet others are partial agonists (e.g. nalorphine). Whilst an opioid partial agonist such as nalorphine, (the N-alkyl analogue of morphine) will antagonize the analgesic effects of morphine, when given alone it can be a potent analgesic in its own right.

Of all of the opioid analgesics, morphine remains the most widely used, but, in addition to its therapeutic properties, it has a number of drawbacks including respiratory depression, decreased gastrointestinal motility (resulting in constipation), nausea and vomiting. Tolerance and physical dependence also limit the clinical uses of opioid compounds.

Aspirin and other salicylate compounds are frequently used in treatment to interrupt amplification of the inflammatory process in rheumatoid diseases and arthritis and temporarily relieve the pain. Other drug compounds used for these purposes include phenylpropionic acid derivatives such as Ibuprofen and Naproxen, Sulindac, phenyl butazone, corticosteroids, antimalarials such as chloroquine and hydroxychloroquine sulfate, and fenemates (J. Hosp. Pharm., 36:622 (May 1979)). These compounds, however, are ineffective for neuropathic pain.

Available therapies for pain also have drawbacks. Some therapeutic agents require prolonged use before an effect is experienced by the patient. Other existing drugs have serious side effects in certain patients, and subjects must be carefully monitored to ensure that any side effects are not unduly threatening. Most existing drugs provide only temporary relief from pain and must be taken consistently on a daily or weekly basis. With disease progression the amount of medication needed to alleviate the pain often increases, thus increasing the potential for adverse side effects.

NMDA receptors are defined by the binding of N-methyl-D-aspartate (NMDA) comprise a receptor/ion channel complex with several different identified binding domains. NMDA itself is a molecule structurally similar to glutamate (Glu) which binds at the glutamate binding suite and is highly selective and potent in activating the NMDA receptor (Watkins (1987); Olney (1989)).

Many compounds are known that bind at the NMDA/Glu binding site (for example CPP, DCPP-ene, CGP 40116, CGP 37849, CGS 19755, NPC 12626, NPC 17742, D-AP5, D-AP7, CGP 39551, CGP-43487, MDL-100,452, LY-274614, LY-233536, and LY233053). Other compounds, referred to as non-competitive NMDA antagonists, bind at other sites in the NMDA receptor complex (examples are phencyclidine, dizocilpine, ketamine, tiletamine, CNS 1102, dextromethorphan, memantine, kynurenic acid, CNQX, DNQX, 6,7-DCQX, 6,7-DCHQC, R(+)-HA-966,7-chloro-kynurenic acid, 5,7-DCKA, 5-iodo-7-chloro-kynurenic acid, MDL-28,469, MDL-100,748, MDL-29,951, L-689,560, L-687,414, ACPC, ACPCM, ACPCE, arcaine, diethylenetriamine, 1,10-diaminodecane, 1,12-diaminododecane, ifenprodil, and SL-82.0715). These compounds have been extensively reviewed by Rogawski (1992) and Massieu et. al., (1993), and articles cited therein.

In addition to its physiological function, glutamate (Glu) can be neurotoxic. Glu neurotoxicity is referred to as "excitotoxicity" because the neurotoxic action of Glu, like its beneficial actions, is mediated by an excitatory process (Olney (1990); Choi (1992)). Normally, when Glu is released at a synaptic receptor, it binds only transiently and is then rapidly removed from the receptor by a process that transports it back into the cell. Under certain abnormal conditions, including stroke, epilepsy and CNS trauma, Glu uptake fails and Glu accumulates at the receptor resulting in a persistent excitation of electrochemical activity that leads to the death of neurons that have Glu receptors. Many neurons in the CNS have Glu receptors, so excitotoxicity can cause an enormous amount of CNS damage.

Acute excitotoxicity injury can occur as a result of ischemic events, hypoxic events, trauma to the brain or spinal cord, certain types of food poisoning which involve an excitotoxic poison such as domoic acid, and seizure-mediated neuronal degeneration, which can result from persistent epileptic seizure activity (status epilepticus). A large body of evidence has implicated the NMDA receptor as one receptor subtype through which Glu mediates a substantial amount of CNS injury, and it is well established that NMDA antagonists are effective in protecting CNS neurons against excitotoxic degeneration in these acute CNS injury syndromes (Choi (1988); Olney (1990)).

In addition to neuronal damage caused by acute insults, excessive activation of Glu receptors may also contribute to more gradual neurodegenerative processes leading to cell death in various chronic neurodegenerative diseases, including Alzheimer's disease, amyotrophic lateral sclerosis, AIDS dementia, Parkinson's disease and Huntington's disease (Olney (1990)). It is generally considered that NMDA antagonists may prove useful in the therapeutic management of such chronic diseases.

In the 1980's it was discovered that PCP (also known as "angel dust") acts at a "PCP recognition site" within the ion channel of the NMDA Glu receptor. PCP acts as a non-competitive antagonist that blocks the flow of ions through the NMDA ion channel. More recently it has become evident that drugs which act at the PCP site as non-competitive NMDA antagonists are likely to have psychotomimetic side effects. Further, it is now recognized that certain competitive and non-competitive NMDA antagonists can cause similar pathomorphological effects in rat brain (Olney et. al., (1991); Hargreaves et. al., (1993)). Such compounds also have psychotomimetic effects in humans (Kristensen et. al., (1992); Herrling (1994); Grotta (1994)).

The glycine binding site of the NMDA receptor complex is distinguishable from the Glu and PCP binding sites. Also, it has recently been discovered that NMDA receptors occur as several subtypes which are characterized by differential properties of the glycine binding site of the receptor. Many compounds that bind at the NMDA receptor glycine site, useful for the treatment of stroke and neurodegenerative conditions, have been described in U.S. Patents 5,604,227; 5,733,910; 5,599,814; 5,593,133; 5,744,471; 5,837,705 and 6,103,721.

## Summary of the invention

It has now been discovered that certain compounds which exhibit the property of binding to the NMDA receptor glycine site have utility for the amelioration of pain and particularly for the amelioration of neuropathic pain.

Therefore, in an aspect the invention provides the following compounds useful for the treatment of pain
7-chloro-4-hydroxy-2-(4-dimethylcarbamoylbenzyl)-1,2,5,10-tetrahydropyridazino[4,5-*b*]quinoline-1,10-dione,
7-chloro-4-hydroxy-2-[3-methyl-4-(pyridin-2-ylmethoxy)-benzyl]-1,2,5,10-tetrahydropyridazino[4,5-*b*]quinoline-1,10-dione,
7-chloro-4-hydroxy-2-(4-dimethylcarbamoyl(3-methyl)benzyl)-1,2,5,10-tetrahydropyridazino[4,5-*b*]quinoline-1,10-dione,
dimethyl-carbamic acid 3-(7-chloro-4-hydroxy-1,10-dioxo-5,10-dihydro-1*H*-pyridazino[4,5-*b*]quinolin-2-ylmethyl)-phenyl ester,
methyl-phenyl-carbamic acid 4-(7-chloro-4-hydroxy-1,10-dioxo-5,10-dihydro-1*H*-pyridazino[4,5-*b*]quinolin-2-ylmethyl)-phenyl ester,
diethyl-carbamic acid 4-(7-chloro-4-hydroxy-1,10-dioxo-5,10-dihydro-1*H*-pyridazino[4,5-*b*]quinolin-2-ylmethyl)-phenyl ester,
diethyl-carbamic acid 3-(7-chloro-4-hydroxy-1,10-dioxo-5,10-dihydro-1*H*-pyridazino[4,5-*b*]quinolin-2-ylmethyl)-phenyl ester,
morpholine-4-carboxylic acid 3-(7-chloro-4-hydroxy-1,10-dioxo-5,10-dihydro-1*H*-pyridazino[4,5-*b*]quinolin-2-ylmethyl)-phenyl ester,
methyl-phenyl-carbamic acid 3-(7-chloro-4-hydroxy-1,10-dioxo-5,10-dihydro-1*H*-pyridazino[4,5-*b*]quinolin-2-ylmethyl)-phenyl ester,
diphenyl-carbamic acid 3-(7-chloro-4-hydroxy-1,10-dioxo-5,10-dihydro-1*H*-pyridazino[4,5-*b*]quinolin-2-ylmethyl)-phenyl ester,
pyrrolidine-1-carboxylic acid 3-(7-chloro-4-hydroxy-1,10-dioxo-5,10-dihydro-1*H*-pyridazino[4,5-*b*]quinolin-2-ylmethyl)-phenyl ester,
morpholine-4-carboxylic acid 4-(7-chloro-4-hydroxy-1,10-dioxo-5,10-dihydro-1*H*-pyridazino[4,5-*b*]quinolin-2-ylmethyl)-2-methyl-phenyl ester.
methyl-phenyl-carbamic acid 4-(7-chloro-4-hydroxy-1,10-dioxo-5,10-dihydro-1*H*-pyridazino[4,5-*b*]quinolin-2-ylmethyl)-2-methyl-phenyl ester,
dimethyl-carbamic acid 5-(7-chloro-4-hydroxy-1,10-dioxo-5,10-dihydro-1*H*-pyridazino[4,5-*b*]quinolin-2-ylmethyl)-2-methoxy-phenyl ester,
diethyl-carbamic acid 5-(7-chloro-4-hydroxy-1,10-dioxo-5,10-dihydro-1*H*-pyridazino[4,5-*b*]quinolin-2-ylmethyl)-2-methoxy-phenyl ester,
morpholine-4-carboxylic acid 5-(7-chloro-4-hydroxy-1,10-dioxo-5,10-dihydro-1*H*-pyridazino[4,5-*b*]quinolin-2-ylmethyl)-2-methoxy-phenyl ester, and
7-chloro-4-hydroxy-2-[4-ethoxycarbonyloyl-3,5-dimethoxybenzyl]-1,2,5,10-tetrahydropyridazino[4,5-*b*]quinoline-1,10-dione,
and pharmaceutically-acceptable salts thereof.
Another aspect of the invention provides the use of said compounds in the manufacture of a medicament for the treatment of neuropathic pain.

Another aspect of the invention is a process for making compounds of the invention, which process comprises the following steps:

a) Preparing a Boc-protected hydrazine according to one of the procedures shown in the following scheme:

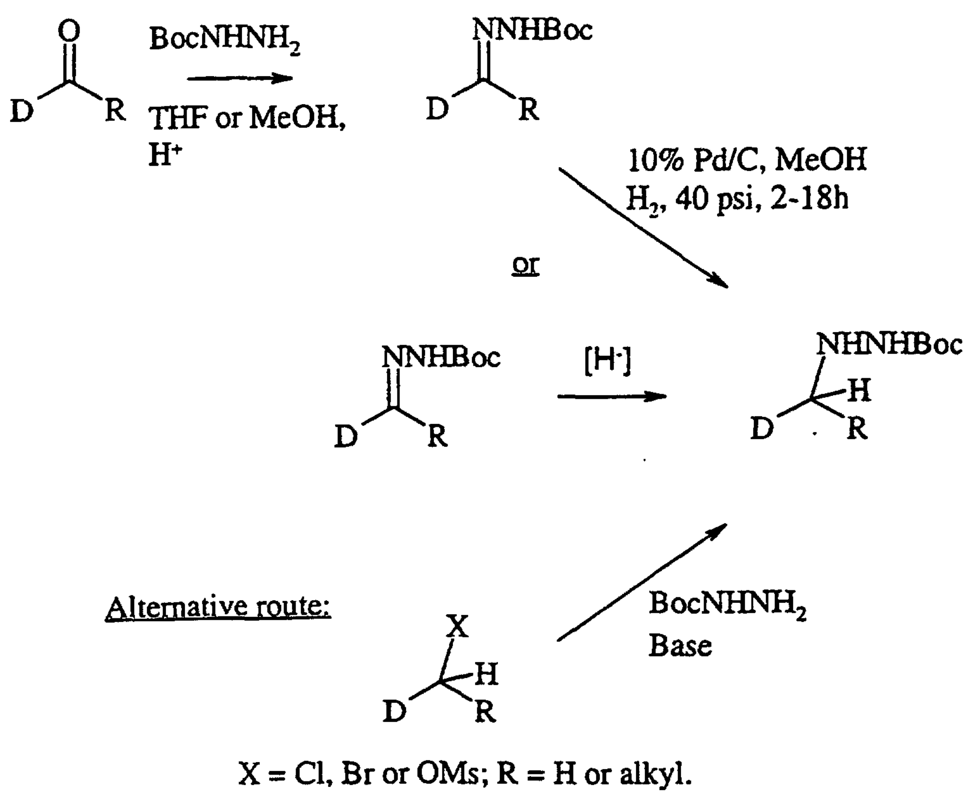


b) coupling said Boc-protected hydrazine and cyclizing the product according to the process of the following scheme to form a compound according to structural diagram I:

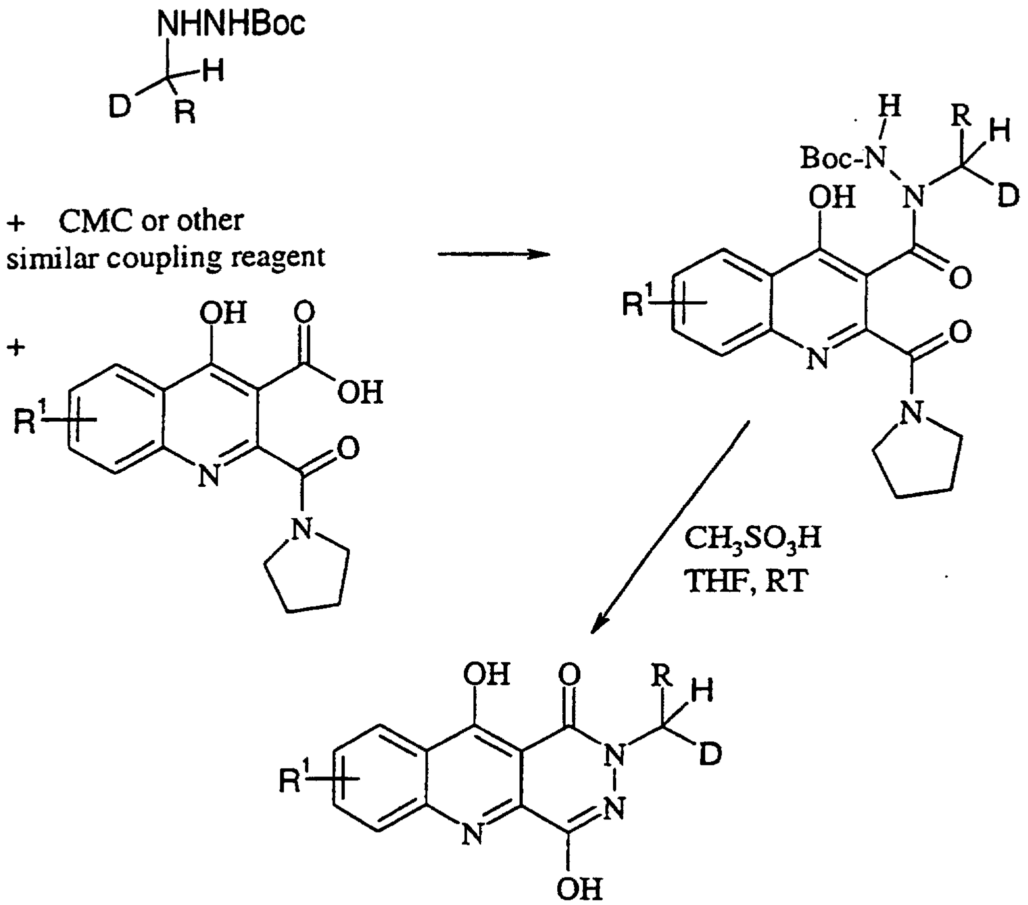


wherein:

CMC is 1-cyclohexyl-3-(2-morpholinoethyl)carbodiimide metho-p-toluenesulfonate;
the "R/H/D" group is the "-A-D" moiety of structural diagram I, and
throughout the foregoing process, $R^1$ is as defined for structural diagram I.

Other compounds within the scope of structural diagram I wherein moiety D thereof is substituted with a carbamate moiety, may be prepared according to the following scheme:

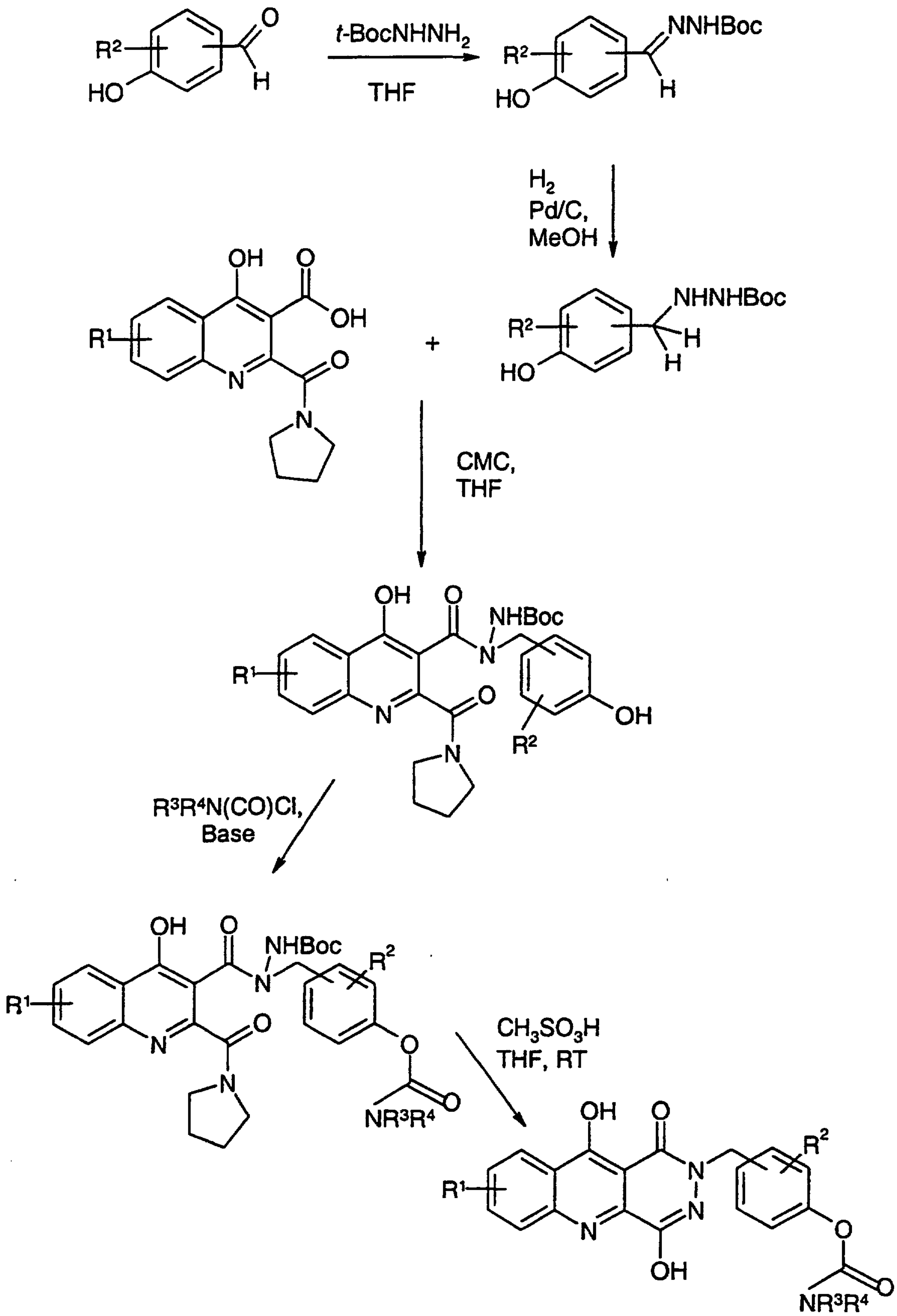


wherein $R^2$, $R^3$ and $R^4$ may be any moiety as defined in structural diagram I.

To use a compound of the invention or a pharmaceutically-acceptable salt thereof for the therapeutic treatment, which may include prophylactic treatment, of pain in mammals, which may be humans, the compound can be formulated in accordance with standard pharmaceutical practice as a pharmaceutical composition.

Suitable pharmaceutical compositions that contain a compound of the invention may be administered in conventional ways, for example by oral, topical, parenteral, buccal, nasal, vaginal or rectal administration or by inhalation. For these purposes a compound of the invention may be formulated by means known in the art into the form of, for example, tablets, capsules, aqueous or oily solutions, suspensions, emulsions, creams, ointments, gels, nasal sprays, suppositories, finely divided powders or aerosols for inhalation, and for parenteral use (including intravenous, intramuscular or infusion) sterile aqueous or oily solutions or suspensions or sterile emulsions. A preferred route of administration is orally by tablet or capsule.

In addition to a compound of the present invention a pharmaceutical composition of this invention may also contain one or more other pharmacologically-active agents, or such pharmaceutical composition may be simultaneously or sequentially co-administered with one or more other pharmacologically-active agents.

Pharmaceutical compositions of this invention will normally be administered so that a pain-ameliorating effective daily dose is received by the subject. The daily dose may be given in divided doses as necessary, the precise amount of the compound received and the route of administration depending on the weight, age and sex of the patient being treated and on the particular disease condition being treated according to principles known in the art. A preferred dosage regime is once daily.

A further embodiment of the invention provides a pharmaceutical composition which contains a compound of the structural diagram I as defined herein or a pharmaceutically-acceptable salt thereof, in association with a pharmaceutically-acceptable additive such as an excipient or carrier.

A yet further embodiment of the invention provide the use of a compound of the structural diagram I, or a pharmaceutically-acceptable salt thereof, in the manufacture of a medicament useful for binding to the NMDA receptor glycine site in a warm-blooded animal such as a human being.

Still another embodiment of the invention provides a method of binding a compound of the invention to the NMDA receptor glycine site of a warm-blooded animal, such as a human being, in need of treatment for pain, which method comprises administering to said animal an effective amount of a compound of structural diagram I or a pharmaceutically-acceptable salt thereof.

Definitions:

When used herein the term "alkyl" includes both straight and branched chain alkyl groups but references to individual alkyl groups such as "propyl" refer to the straight chain moiety.

When used herein the term "halo" means fluoro, chloro, bromo and iodo.

When used herein the term "aryl" means an unsaturated carbon ring or a benz-derivative thereof. Particularly, aryl means phenyl, naphthyl or biphenyl. More particularly aryl means phenyl.

When used herein the term "heteroaryl" or "heteroaryl ring" means, unless otherwise further specified, a monocyclic-, bicyclic- or tricyclic- 5-14 membered ring that is unsaturated or partially unsaturated, with up to five ring heteroatoms selected from nitrogen, oxygen and sulphur wherein a $-CH_2-$ group can optionally be replaced by a -C(O)-, and a ring nitrogen atom may be optionally oxidized to form the N-oxide. Examples of such heteroaryls include thienyl, furyl, pyranyl, pyrrolyl, imidazolyl, pyrazolyl, thiazolyl, oxazolyl, isoxazolyl, pyridyl, pyridyl-*N*-oxide, oxopyridyl, oxoquinolyl, pyrimidinyl, pyrazinyl, oxopyrazinyl, pyridazinyl, indolinyl, benzofuranyl, benzimidazolyl, benzothiazolyl, quinolyl, isoquinolinyl, quinazolinyl, xanthenyl, quinoxalinyl, indazolyl, benzofuranyl and cinnolinolyl.

When used herein the term "heterocyclyl" or "heterocyclic ring" means, unless otherwise further specified, a mono- or bicyclic- 5-14 membered ring, that is totally saturated, with up to five ring heteroatoms selected from nitrogen, oxygen and sulphur wherein a $-CH_2-$group can optionally be replaced by a -C(O)-. Examples of such heterocycles include morpholinyl, pyrrolidinyl, imidazolidinyl, pyrazolidinyl, piperidinyl, piperazinyl, homopiperidinyl, homopiperazinyl and quinuclidinyl.

When used herein, where optional substituents are selected from "one or more" groups it is to be understood that this encompasses compounds where all substituents are chosen from one of the specified groups and compounds where substituents are chosen from more than one of the specified groups.

Generally in the methods, processes and examples described herein:

concentrations were carried out by rotary evaporation *in vacuo;*
operations were carried out at ambient temperature, that is in the range 18-26 °C and under a nitrogen atmosphere;
column chromatography (by the flash procedure) was performed on Merck Kieselgel silica (Art. 9385) unless otherwise stated;
yields are given for illustration only and are not necessarily the maximum attainable;
the structure of the end-products of the formula I were generally confirmed by NMR and mass spectral techniques,

proton magnetic resonance spectra were determined in DMSO-$d_6$ unless otherwise stated using a Varian Gemini 2000 spectrometer operating at a field strength of 300 MHz; chemical shifts are reported in parts per million downfield from tetramethylsilane as an internal standard (δ scale) and peak multiplicities are shown thus: s, singlet; bs, broad singlet; d, doublet; AB or dd, doublet of doublets; t, triplet, dt, double of triplets, m, multiplet; bm, broad multiplet; fast-atom bombardment (FAB) mass spectral data were obtained using a Platform spectrometer (supplied by Micromass) run in electrospray and, where appropriate, either positive ion data or negative ion data were collected, in this application, $(M+H)^+$ is quoted; IR data was obtained with a Nicolet Avatar 360 FT-IR;

intermediates were not generally fully characterized and purity was in general assessed mass spectral (MS) or NMR analysis.

The following abbreviations and definitions when used, have the meanings, as follows:

| | |
|---|---|
| $CDCl_3$ | is deuterated chloroform; |
| CMC | is 1-cyclohexyl-3-(2-morpholinoethyl)carbodiimide metho-*p*-toluenesulfonate; |
| DCM | is dichloromethane; |
| DCU | is dicyclohexyl urea; |
| DHC | is 1,3-dicyclohexylcarbodiimide; |
| DMAP | is 4-(dimethylamino)pyridine; |
| DMF | is N,N-dimethylformamide; |
| DMSO | is dimethylsulphoxide; |
| m/s | is mass spectroscopy; |
| NMP | is N-methylpyrrolidinone; |
| NMR | is nuclear magnetic resonance; |
| p.o. | is *per os;* |
| THF | is tetrahydrofuran, and |
| t.i.d. | is three times daily. |

The examples and tests described herein are intended to illustrate but not limit the invention.

## Examples:

**Example 1:** 7-Chloro-4-hydroxy-2-(4-dimethylcarbamoylbenzyl)-1,2,5,10-tetrahydropyridazino[4,5-*b*]quinoline-1,10-dione.

*N'*-(4-Hydroxy-benzylidene)-hydrazinecarboxylic acid *tert*-butyl ester.

To a stirred solution of 4-hydroxybenzaldehyde (2.5 g, 20.0 mmol) in THF (80 mL) was added *tert*-butyl carbazate (2.72 g, 20.5 mmol) and concentrated hydrochloric acid, approximately 2 drops from a Pasteur pipette. The solution was stirred overnight at room temperature and the volatiles removed under reduced pressure. The resultant solid was triturated with hexanes and collected to give the title compound as a tan solid (4.7 g, 100%). $^1H$ NMR (300 MHz, DMSO-$d_6$): δ 1.45 (s, 9H); 6.77 (d, 2H, J = 8.7 Hz); 7.40 (d, 2H, J = 8.7 Hz); 7.88 (br s, 1H); 9.78 (s, 1H); 10.66 (br s, 1H).

*N'*-(4-Dimethylcarbamoyloxy-benzylidene)-hydrazinecarboxylic acid *tert*-butyl ester.

To a slurry of *N'*-(4-hydroxy-benzylidene)-hydrazinecarboxylic acid *tert*-butyl ester (1.0 g, 4.23 mmol) in anhydrous pyridine (5 mL) was slowly added *N*,*N*-dimethylcarbamoyl chloride (0.45 g, 0.39 mL, 4.23 mmol). The mixture was refluxed for 5 hours and then cooled to room temperature. Over time, a solid fell out of solution. The solid was collected, washed with water (20 mL) and then diethyl ether (20 mL). This material was then dried under reduced pressure to give the title compound as a cream colored-solid (1.01 g, 76%). $^1H$ NMR (300 MHz, DMSO-$d_6$): δ 1.46 (s, 9H); 2.91 (s, 3H); 3.04 (s, 3H); 7.15 (d, 2H, J = 8.7 Hz); 7.55 (d, 2H, J = 8.7 Hz); 7.98 (br s, 1H); 10.89 (br s, 1H).

*N'*-(4-Dimethylcarbamoyloxy-benzyl)-hydrazinecarboxylic acid *tert*-butyl ester.

*N'*-(4-Dimethylcarbamoyloxy-benzylidene)-hydrazinecarboxylic acid *tert*-butyl ester (1.0 g, 3.25 mmol) was dissolved in methyl alcohol (60 mL) and placed in a Parr shaker bottle. To this was added 10% palladium-on-carbon (250 mg) and the reaction was hydrogenated at 40 psi for 6 hours. The catalyst was filtered on diatomaceous earth, washed with methyl alcohol (2 x 100 mL), and the solvents were removed *in vacuo* to give a white solid (0.62 g, 62%). $^1H$

NMR (300 MHz, DMSO-$d_6$): δ 1.38 (s, 9H); 2.84 (s, 3H); 3.03 (s, 3H); 3.84 (d, 2H, J = 3.9 Hz); 4.75 (m, 1H); 7.06 (d, 2H, J = 8.7 Hz); 7.26 (d, 2H, J = 8.7 Hz); 8.25 (br s, 1H).

Dimethyl 7-chloro-4-hydroxyquinoline-2,3-dicarboxylate:

A stirred mixture of methyl 2-amino-4-chlorobenzoate (2.50 g, 13.5 mmol) and dimethyl acetylenedicarboxylate (2.05 g, 14.4 mmol) in *tert*-butanol (22 ml) was refluxed for 7 hours under a nitrogen atmosphere. After adding additional dimethyl acetylenedicarboxylate (1.16 g, 8.13 mmol) and refluxing another 2.5 hours, the reaction mixture was allowed to cool to room temperature and potassium *tert*-butoxide (1.56 g, 13.9 mmol) was added in one portion. A precipitate formed and the resulting mixture was refluxed for 1.5 hours. The mixture was cooled to room temperature and filtered to separate the solids, which were washed with *tert*-butanol and diethyl ether. The solids were dissolved in water and acidified with 1 N sulfuric acid to form a precipitate. The resulting mixture was extracted with DCM and the combined extracts were washed with brine and water, dried over $MgSO_4$, filtered and concentrated to give a green solid. Recrystallization of this material from methanol provided the title compound (1.15 g, 47%) as an off-white solid, mp 232-233 °C; MS (CI):296 (M+H). Analysis for $C_{13}H_{10}ClNO_5$: Calc'd: C, 52.81; H, 3.41; N, 4.74; Found: C, 52.75; H, 3.47; N, 4.69.

3-Carbomethoxy-7-chloro-4-hydroxyquinoline-2-carboxylic acid:

To a stirred suspension of dimethyl 7-chloro-4-hydroxyquinoline-2,3-dicarboxylate (1.0 g, 3.38 mmol) in water (20 mL) was added an aqueous solution of sodium hydroxide (0.27 g, 6.75 mmol). Upon addition, the suspension dissolved. The reaction mixture was warmed to 60 °C for 1 hour. After this time the reaction was cooled to room temperature and acidified with concentrated hydrochloric acid. The product was then extracted into diethyl ether and ethyl acetate. The organic extracts were dried over $MgSO_4$, filtered and concentrated *in vacuo* to provide the title compound as a solid (900 mg). This material was purified by recrystallization employing an ethyl acetate/hexane co-solvent system to provide the title compound (571 mg, 60%) as a white solid mp 296 °C (dec); MS (CI) = 238 (M+H). Analysis for $C_{12}H_8NO_5Cl$•0.45 $CH_3CO_2CH_2CH_3$• 0.10 $H_2O$: Calc'd: C, 51.30; H, 3.68; N 4.34, Found: C, 51.28; H, 3.62; N 3.97 $^1H$ NMR 8.22 (d, J = 8.7 Hz, 1H), 7.92 (d, J = 1.8 Hz, 1H), 7.28 (dd, J = 8.7, 1.8 Hz, 1H), 3.90 (s, 3H).

3-Carbomethoxy-2-pyrrolidinocarbamide-7-chloro-4-hydroxyquinoline:

To a suspension of 3-carbomethoxy-7-chloro-4-hydroxyquinoline-2-carboxylic acid (2.25 g, 8.0 mmol) in THF (20 mL) at ambient temperature under a $N_2$ atmosphere was added DHC (1.65 g, 8.0 mmol) and pyrrolidine (0.596 g, 8.4 mmol). The reaction was stirred room temperature for 15 hours after which time the by-product urea was removed via filtration. The desired product was purified via flash column chromatography employing 5% methanol in chloroform to provide the title compound (2.52 g, 94.3%) as a tan solid, mp = 215 °C; MS (CI): 335 (M+H). 300 MHz $^1H$ NMR (DMSO-$d_6$): δ 8.12 (d, J = 8.7 Hz, 1H), 7.60 (d, 1H, J = 1.8 Hz), 7.47 (dd, 1H, J = 8.8, 2.0 Hz), 3.69 (s, 3H), 3.40-3.49 (m, 2H), 3.27-3.33 (m, 2H), 1.80-1.96 (m, 4H).

7-Chloro-4-oxo-2-(pyrrolidinylcarbonyl)hydroquinoline-3-carboxylic acid:

To a suspension of 3-carbomethoxy-2-pyrrolidinocarbamide-7-chloro-4-hydroxy quinoline (2.52g, 7.5 mmol) in de-ionized water (40 mL) was added dropwise a solution (20 mL) of an aqueous potassium hydroxide (882 mg, 15.75 mmol). Upon complete addition, the reaction was warmed to 60 °C. After 3 hours, the reaction was filtered to remove a small amount of insoluble material. The filtrate was then acidified to pH = 1 which yield a white precipitate. The solid was isolated by vacuum filtration, washed with water, and dried at 30 °C *in vacuo* for 16 hours. This provided the title compound (1.5 g, 64%) as a white solid, mp = 225-8 °C; MS (CI): 321 (M+H). 300 MHz $^1H$ NMR (DMSO-$d_6$): δ 8.28 (d, J = 8.8 Hz, 1H), 7.77 (s, 1H), 7.64 (d, 1H, J = 8.7), 3.52-3.57 (m, 2H), 3.17-3.19 (m, 2H), 1.83-1.98 (m.4H).

*N'*-[7-Chloro-4-oxo-2-(pyrrolidine-1-carbonyl)-1,4-dihydroquinoline-3-carbonyl]-*N'*-(4-dimethylcarbamoyloxy-benzyl)-hydrazinecarboxylic acid *tert*-butyl ester.

To a stirred slurry of 7-chloro-4-oxo-2-(pyrrolidinylcarbonyl)hydroquinoline-3-carboxylic acid (0.53 g, 1.67 mmol) in THF (30 mL) was added CMC (0.92 g, 2.17 mmol) and the reaction was stirred for five minutes. To this mixture was added, via dropwise addition, a solution of *N'*-(4-dimethylcarbamoyloxy-benzyl)-hydrazinecarboxylic acid *tert*-butyl ester (0.62 g, 2.00 mmol) and DMAP (ca. 0.005 g) in THF (10 mL). The mixture was then refluxed overnight. The solution was filtered, washed with THF (15 mL) and the insolubles collected. The insoluble material was then washed with water (100 mL) and THF (20 mL). The remaining insoluble material was dried *in vacuo* to give the title compound

as a white solid (0.785 g, 76%). The material was used in the following reaction without further purification.

7-Chloro-4-hydroxy-2-(4-dimethylcarbamoylbenzyl)-1,2,5,10-tetrahydropyridazino[4,5-*b*]quinoline-1,10-dione.

To a stirred solution of *N*'-[7-chloro-4-oxo-2-(pyrrolidine-1-carbonyl)-1,4-dihydroquinoline-3-carbonyl] -*N*'-(4-dimethylcarbamoyloxy-benzyl)-hydrazinecarboxylic acid *tert*-butyl ester (0.78 g, 0.33 mmol) in THF (10 mL) was added methanesulfonic acid (2.88 mL) and the reaction was heated to 70 °C for 15 minutes. The mixture was cooled to room temperature and stirred for 4 hours. The THF was removed under reduced pressure to yield an orange oil. To this was added diethyl ether (50 mL) and the reaction stirred for 10 minutes. The diethyl ether was carefully decanted to leave a thick orange oil. To this was added water (15 mL) and a fluffy yellow precipitate formed. The mixture was stirred for 10 minutes and then filtered. The solids were washed with water (10 mL) followed by diethyl ether (20 mL). The solids were collected and sonicated in methyl alcohol/diethyl ether (1:1, 20 mL) for 15 minutes. The solids were collected and dried under reduced pressure to give the title compound as a cream-colored solid (0.30 g, 55%). $^1$H NMR (300 MHz, DMSO-$d_6$): δ 2.89 (s, 3H); 3.02 (s. 3H); 5.10 (s, 2H); 7.03 (d, 2H, J = 8.7 Hz); 7.29 (d, 2H, J = 8.7 Hz); 7.42 (d, 1H, J = 8.7 Hz); 8.01 (s, 1H); 8.14 (d, 1H, J= 8.7 Hz); 11.92 (s, 1H); 12.65 (s, 1H). Calc'd. for $C_{21}H_{17}ClN_4O_5$•0.4 $H_2O$: C, 56.29; H, 4.00; N, 12.50. Found: C, 56.46; H, 3.84; N, 12.38.

**Example 2:** 7-Chloro-4-hydroxy-2-[3-methyl-4-(pyridin-2-ylmethoxy)-benzyl]-1,2,5,10-tetrahydropyridazino[4,5-*b*] quinoline-1,10-dione.

N-[1-Aza-2-(4-hydroxy-3-methylphenyl)vinyl](*tert*-butoxy)carboxamide

4-Hydroxy-3-methylbenzaldehyde (2.0 g, 14.6 mmol) was added to THF (60 mL) and to this was added *tert*-butylcarbazate (2.03 g, 15.4 mmol). The reaction mixture was stirred for 16 hours and then the solvent was removed under reduced pressure to give the title compound as a white solid (3.5 g, 95%). This material was used in the next reaction without further purification.

(*tert*-Butoxy)-N-{[(4-hydroxy-3-methylphenyl)methyl]amino}carboxamide

N-[1-Aza-2-(4-hydroxy-3-methylphenyl)vinyl](*tert*-butoxy)carboxamide (2.0 g, 7.92 mmol) was added to a Parr shaker bottle and dissolved in methyl alcohol (30 mL). To this was added 10% palladium-on-carbon (~300 mg) and the reaction was hydrogenated at 40 psi for 3 hours. The catalyst was removed by filtration through diatomaceous earth, washed with methyl alcohol (2 x 100 mL), and the solvents were removed *in vacuo* to yield an oil. The oil was dissolved in DCM, and the solvent removed *in vacuo* to give the title compound as an oil (1.89 g, 95%). $^1$H NMR (300 MHz, DMSO-$d_6$): δ 1.38 (s, 9H); 2.08 (s, 3H); 3.62 (s, 2H); 4.40 (br s, 1H); 6.65 (d, 1H, J = 8.1 Hz); 6.88 (dd, 1H, J = 1.8, 8.1 Hz); 6.98 (s, 1H); 8.17 (br s, 1H); 9.11 (s, 1H).

N-[(*tert*-Butoxy)carbonylamino][7-chloro-4-oxo-2-(pyrrolidinylcarbonyl)(3-hydroquinolyl)]-N-[(4-hydroxy-3-methylphenyl)methyl]carboxamide.

To a stirred slurry of 7-chloro-4-oxo-2-(pyrrolidinylcarbonyl)hydroquinoline-3-carboxylic acid, Example 1, (1.89 g, 5.91 mmol) in THF (50mL) was added CMC (2.99 g, 7.09 mmol) and the reaction was stirred for five minutes. To this mixture was added, via dropwise addition, a solution of (*tert*-butoxy)-N-{[(4-hydroxy-3-methylphenyl)methyl]amino} carboxamide (1.61 g, 6.38 mmol) and DMAP (0.200 g, 1.63 mmol) in THF (5 mL), and the mixture was then refluxed for 8 hours. The solution was filtered and the insolubles washed with THF (2 x 20 mL). The THF was evaporated and the residual solid chromatographed ($SiO_2$, 95/5 chloroform/diethyl ether) to give the title compound as a yellow foam (2.1 g, 65%). This material was used in the next reaction without further purification.

7-Chloro-4-hydroxy-2-[3-methyl-4-(pyridin-2-ylmethoxy)-benzyl]-1,2,5,10-tetrahydropyridazino[4,5-*b*]quinoline-1,10-dione.

To a stirred solution of N-[(*tert*-butoxy)carbonylamino][7-chloro-4-oxo-2-(pyrrolidinylcarbonyl)(3-hydroquinolyl)]-N-[(4-hydroxy-3-methylphenyl)methyl]carboxamide (0.30 g, 0.53 mmol) in DMF (5 mL) was added 2-picolyl chloride hydrochloride (0.11 g, 0.66 mmol) followed by potassium carbonate (0.500 g, 3.61 mmol). The mixture was heated to 100 °C for 8 hours. The solution was cooled to room temperature and diluted with DCM (50 mL). The organic layer was extracted with water (2 x 30 mL), brine (1 x 30 mL) and dried over $Na_2SO_4$. The volatiles were removed under reduced pressure to give a dark yellow oil. MS (-CI): *m/z* 644/647. The oil was subsequently dissolved in THF (5 mL) and to this was added methanesulfonic acid (1 mL). The mixture was stirred for 16 hours and the volatiles removed

under reduced pressure. The residual oil was triturated with diethyl ether (10 mL) for ten minutes and then the ether layer was carefully decanted away from the oil. The oil was then treated with water (5 mL) which induced precipitation to occur. The solids were collected by filtration, and washed with diethyl ether. The material was then sonicated in diethyl ether/methyl alcohol (4:1, 5 mL, 5 minutes) and filtered to give a brown solid (0.006 g, 3% yield for two steps). $^1$H NMR (300 MHz, DMSO-$d_6$): δ 2.21 (s, 3H); 2.30 (s, 3H); 5.00 (s, 2H); 5.21 (s, 2H); 6.93 (d, 1H, J = 8.7 Hz); 7.09 (d, 1H, J = 8.7 Hz); 7.14 (s, 1H); 7.43 (m, 2H); 7.59 (d, 1H, J = 7.8 Hz); 7.95 (app t, 1H, J = 7.8 Hz); 8.01 (s, 1H); 8.14 (d, 1H, J = 8.7 Hz); 8.61 (d, 1H, J = 4.8 Hz); 11.95 (br s, 1H); 12. 53 (br s). MS (-CI): *m/z* 473/474.

**Example 3:** 7-Chloro-4-hydroxy-2-(4-dimethylcarbamoyl(3-methyl)benzyl)-1,2,5,10-tetrahydropyridazino[4,5-*b*]quinoline-1,10-dione.

*N*'-(4-Hydroxy-3-methyl-benzylidene)-hydrazinecarboxylic acid *tert*-butyl ester.

To a stirred solution of 4-hydroxy-3-methylbenzaldehyde (2.0 g, 14.6 mmol) in THF (80 mL) was-added *tert*-butyl carbazate (2.03 g, 15.4 mmol). The solution was stirred for 16 hours at room temperature and the volatiles removed under reduced pressure. The resultant solid was triturated with hexanes and collected to give the title compound as a tan solid (3.5 g, 95%). The material was used without further purification in the following reaction.

*N*'-(4-Hydroxy-3-methyl-benzyl)-hydrazinecarboxylic acid *tert*-butyl ester.

*N*'-(4-Hydroxy-3-methyl-benzylidene)-hydrazinecarboxylic acid *tert*-butyl ester (2.0 g, 7.99 mmol) was dissolved in methyl alcohol (60 mL) and placed in a Parr shaker bottle. To this was added 10% palladium-on-carbon (250 mg) and the reaction was hydrogenated at 40 psi for 4 hours. The catalyst was filtered on diatomaceous earth, washed with methyl alcohol (2 x 100 mL), and the solvents were removed *in vacuo* to give a white solid (1.89 g, 94%). $^1$H NMR (300 MHz, DMSO-$d_6$): δ 1.38 (s, 9H); 2.08 (s, 3H); 3.62 (br s, 2H); 4.40 (br s, 1H); 6.65 (s, 1H, J = 8.1 Hz); 6.89 (dd, 1H J = 1.8 Hz, 8.1 Hz); 6.98 (s, 1H); 8.17 (br s, 1H).

*N*'-[7-Chloro-4-oxo-2-(pyrrolidine-1-carbonyl)-1,4-dihydroquinoline-3-carbonyl]-*N*'-(4-hydroxy-3-methyl-benzyl)-hydrazinecarboxylic acid *tert*-butyl ester.

To a stirred slurry of 7-chloro-4-oxo-2-(pyrrolidinylcarbonyl)hydroquinoline-3-carboxylic acid, Example 1, (1.89 g, 5.91 mmol) in THF (50 mL) was added CMC (2.99 g, 7.09 mmol) and the reaction was stirred for five minutes. To this mixture was added, via dropwise addition, a solution of *N*'-(4-hydroxy-3-methyl-benzyl)-hydrazinecarboxylic acid *tert*-butyl ester (1.61 g, 6.38 mmol) and DMAP (0.08 g, 0.65 mmol) in THF (5 mL), and the mixture was then refluxed for 8 hours. The solution was filtered and the insolubles washed with DCM (2 x 150 mL). The mother liquor was collected and concentrated to dryness. The resultant yellow foam was subjected to chromatography ($SiO_2$, 95/5 chloroform/methyl alcohol) to give the title compound as a yellow foam (2.10 g, 65%). MS (-CI) *m/z* 551/552

*N*'-[7-Chloro-4-oxo-2-(pyrrolidine-1-carbonyl)-1,4-dihydroquinoline-3-carbonyl]-*N*'-(4-dimethylcarbamoyloxy-3-methyl-benzyl)-hydrazinecarboxylic acid *tert*-butyl ester.

To a stirred solution of DCM and *N*'-[7-chloro-4-oxo-2-(pyrrolidine-1-carbonyl)-1,4-dihydroquinoline-3-carbonyl]-*N*-(4-hydroxy-3-methyl-benzyl)-hydrazinecarboxylic acid *tert*-butyl ester (0.35 g, 0.63 mmol) was added triethylamine (0.10 mL, 0.72 mmol) and DMAP (0.03 g, 0.24 mmol) and *N*,*N*-dimethylcarbamoyl chloride (0.07 mL, 0.78 mmol). The solution was stirred for 16 hours and then diluted with DCM (50 mL). The organic layer was extracted with aqueous ammonium chloride (saturated, 1 x 15 mL), aqueous ammonium hydroxide (15 %, 1 x 15 mL), and aqueous sodium chloride (saturated, 1 x 15 mL). The organic layer was then dried over $Na_2SO_4$ and concentrated to dryness. The resultant oil was used without further purification in the following reaction. MS (-CI) *mlz* 624/625

7-Chloro-4-hydroxy-2-(4-dimethylcarbamoyl(3-methyl)benzyl)-1,2,5,10-tetrahydropyridazino[4,5-*b*]quinoline-1,10-dione.

To a stirred solution of *N*'-[7-chloro-4-oxo-2-(pyrrolidine-1-carbonyl)-1,4-dihydroquinoline-3-carbonyl]-*N*'-(4-dimethylcarbamoyloxy-3-methyl-benzyl)-hydrazinecarboxylic acid *tert*-butyl ester (0.25 g, 0.40 mmol) in THF (10 mL) was added methanesulfonic acid (1.2 mL) and the reaction was stirred at room temperature for 16 hours. The THF was removed under reduced pressure to yield an orange oil. To this was added diethyl ether (50 mL) and the reaction stirred for 10 minutes. The ether was carefully decanted to leave a thick orange oil. To this was added water (5 mL) and a fluffy yellow precipitate formed. The mixture was stirred for 10 minutes and then filtered. The solids were

washed with water (10 mL) followed by diethyl ether (20 mL). The solids were collected and sonicated in methyl alcohol/ diethyl ether (1:10, 5 mL) for 10 minutes. The solids were collected and dried under reduced pressure to give the title compound as a cream-colored solid (0.06 g, 30%). $^1$H NMR (300 MHz, DMSO-$d_6$): δ 2.09 (s, 3H); 2.89 (s, 3H); 3.05 (s, 3H); 5.07 (s, 2H); 6.98 (d, 1H, J = 8.1 Hz); 7.13 (d, 1H, J = 8.1 Hz); 7.18 (s, 1H); 7.42 (d, 1H, J = 8.4 Hz); 8.02 (s, 1H); 8.14 (d, 1H, J = 8.4 Hz); 11.93 (br s, 1H); 12. 64 (br s). MS (-CI): *mlz* 453/454.

**Examples 4 -16:**

Table 1 shows carbamate-containing pyridazino quinoline diones of the present invention and the physical characteristics thereof. The compounds were made by the indicated method from commercially available carbamoyl chlorides or hydroxybenzaldehydes

Table 1:

| Ex. | Compound Name | Yield | NMR (300 MHz, DMSO-$d_6$): δ | MS | Method |
|---|---|---|---|---|---|
| **4** | Dimethyl-carbamic acid 3-(7-chloro-4-hydroxy-1,10-dioxo-5,10-dihydro-1*H*-pyridazino[4,5-*b*] quinolin-2-ylmethyl)-phenyl ester. | 48% | 2.88 (s, 3H); 3.01 (s, 3H); 5.11 (s, 2H); 7.02 (m, 2H); 7.13 (d, 1H, J = 7.8 Hz); 7.33 (t, 1H, J = 7.8 Hz); 7.43 (d, 1H, J = 8.7 Hz); 8.02 (s, 1H); 8.15 (d, 1H, J = 8.7 Hz); 11.94 (br s, 1H); 12. 70 (br s, 1H). | (-CI) *m*/*z* 439/441 | 1 |
| **5** | Methyl-phenyl-carbamic acid 4-(7-chloro-4-hydroxy-1,10-dioxo-5,10-dihydro-1*H*-pyridazino[4,5-*b*] quinolin-2-ylmethyl)-phenyl ester. | 40% | 3.39 (s, 3H); 5.10 (s, 2H); 7.09 (d, 2H, J = 8.4 Hz); 7.24 (m, 3H); 7.43 (m, 5H); 8.01 (s, 1H); 8.14 (d, 1H, J = 8.7 Hz); 11.92 (br s, 1H); 12. 66 (br s, 1H). | (-CI) *m*/*z* 501/503 | 1 |
| **6** | Diethyl-carbamic acid 4-(7-chloro-4-hydroxy-1,10-dioxo-5,10-dihydro-1*H*-pyridazino[4,5-*b*] quinolin-2-ylmethyl)-phenyl ester. | 38% | 1.10 (m ,3 H); 1.16 (m, 3H); 3.30 (m, 4H); 5.10 (s, 2H); 7.06 (d, 2H, J = 8.4 Hz); 7.33 (d, 1H, J = 8.4 Hz); 7.42 (d, 1H, J = 8.7 Hz); 8.02 (s, 1H); 8.14 (d, 1H, J = 8.7 Hz); 11.92 (br s, 1H); 12.66 (br s, 1H). | (-CI) *m*/*z* 467/468/ 469 | 1 |
| **7** | Diethyl-carbamic acid 3-(7-chloro-4-hydroxy-1,10-dioxo-5,10-dihydro-1*H*-pyridazino[4,5-*b*] quinolin-2-ylmethyl)-phenyl ester. | 38% | 1.09 (m, 3H); 1. 17 (m, 3H); 3.27 (m, 2H); 3.37 (m, 2H); 5.11 (s, 2H); 7.01 (d, 1H, J = 8.1 Hz); 7.05 (s, 1H); 7.13 (d, 1H, J = 7. 8 Hz); 7.33 (t, 1H, J = 7.8 Hz); 7.43 (d, 1H, J = 8.7 Hz); 8.02 (d, 1H, J = 1.8 Hz); 8.15 (d, 1H , J = 8.7 Hz); 11.94 (br s, 1H); 12.68 (br s, 1H). | (-CI) *m*/*z* 468/469/ 470 | 1 |
| **8** | Morpholine-4-carboxylic acid 3-(7-chloro-4-hydroxy-1,10-dioxo-5,10-dihydro-1*H*-pyridazino[4,5-b] quinolin-2-ylmethyl)-phenyl ester. | 18% | 2.31 (MsOH adduct); 3.39 (br m, 2H); 3.55 (br m, 2H); 3.82 (br m, 4H); 5.11 (s, 2H); 7.04 (m, 2H); 7.15 (d, 1H, J = 7.8 Hz); 7.35 (t, 1H, J = 7.8 Hz); 7.44 (d, 1H, J = 8.7 Hz); 8.03 (s. 1H); 8.15 (d, 1H, J = 8.7 Hz). | (-CI) *m*/*z* 481/482/ 483 | 3 |

Table 1: (continued)

| Ex. | Compound Name | Yield | NMR (300 MHz, DMSO-$d_6$): δ | MS | Method |
|---|---|---|---|---|---|
| **9** | Methyl-phenyl-carbamic acid 3-(7-chloro-4-hydroxy-1,10-dioxo-5,10-dihydro-1H-pyridazino[4,5-*b*] quinolin-2-ylmethyl)-phenyl ester. | 31% | 3.32 (s, 3H); 5.10 (s, 2H); 7.06 (m, 3H); 7.14 (d, 1H, J = 7.8 Hz); 7.30 (m, 2H); 7.43 (m, 4H); 8.02 (s, 1H); 8.14 (d, 1H, J = 8.7 Hz); 11.95 (br s, 1H); 12. 68 (br s, 1H). | (-CI) *m*/*z* 501/502 | 3 |
| **10** | Diphenyl-carbamic acid 3-(7-chloro-4-hydroxy-1,10-dioxo-5,10-dihydro-1*H*-pyridazino[4,5-*b*] quinolin-2-ylmethyl)-phenyl ester. | 16%[2] | 5.10 (s, 2H); 7.13 (m, 3H); 7.27 (m, 2H); 7.40 (m, 10H); 8.02 (s, 1H); 8.14 (d, 1H, J = 8.7 Hz); 11.93 (br s, 1H); 12. 66 (br s, 1H). | (-CI) *m*/*z* 563/564 | 3 |
| **11** | Pyrrolidine-1-carboxylic acid 3-(7-chloro-4-hydroxy-1,10-dioxo-5,10-dihydro-1*H*-pyridazino[4,5-*b*] quinolin-2-ylmethyl)-phenyl ester. | 23% | 1.85 (m, 4H); 3.30 (d, 2H, J = 6.3 Hz); 3.46 (d, 2H, J = 6.3 Hz); 5.11 (s, 2H); 7.04 (m, 1H); 7.12 (m, 1H); 7.36 (t, 1H, J = 7.8 Hz); 7.44 (d, 1H, J = 8.7 Hz); 8.04 (s, 1H); 8.13 (d, 1H, J = 8.7 Hz); 11.92 (br s, 1H); 12.68 (br s, 1H). | (-CI) *m*/*z* 466/467 | 3 |
| **12** | Morpholine-4-carboxylic acid 4-(7-chloro-4-hydroxy-1,10-dioxo-5,10-dihydro-1*H*-pyridazino[4,5-*b*] quinolin-2-ylmethyl)-2-methyl-phenyl ester. | 26% | 2.10 (s, 3H); 3.42 (m, 4H); 3.62 (m, 2H); 7.02 (d, 1H, J = 8.1 Hz); 7.14 (d, 1H, J = 8.1 Hz); 7.19 (s, 1H); 7.44 (d, 1H, J = 8.7 Hz); 8.03 (s, 1H); 8.14 (d, 1H, J = 8.7 Hz); 11.94 (br s, 1H); 12. 64 (br s, 1H). | (-CI) *m*/*z* 495/496 | 3 |
| **13** | Methyl-phenyl-carbamic acid 4-(7-chloro-4-hydroxy-1,10-dioxo-5,10-dihydro-1*H*-pyridazino[4,5-*b*] quinolin-2-ylmethyl)-2-methyl-phenyl ester. | 18% | 2.08 (s, 3H); 3.33 (s, 3H); 7.04 (m, 2H); 7.13 (m, 2H); 7.17 (s, 1H); 7.29 (m, 1H); 7.44 (m, 5H); 8.02 (s, 1H); 8.15 (d, 1H, J = 8.7 Hz); 11.91 (br s, 1H); 12. 63 (br s, 1H). | (-CI) *m*/*z* 514/515 | 3 |
| **14** | Dimethyl-carbamic acid 5-(7-chloro-4-hydroxy-1,10-dioxo-5,10-dihydro-1*H*-pyridazino[4,5-*b*] quinolin-2-ylmethyl)-2-methoxy-phenyl ester. | 41% | 2.86 (s, 3H); 3.00 (s, 3H); 3.73 (s, 3H); 5.03 (s, 2H); 7.02 (m, 2H); 7.15 (d, 1H, J = 8.1 Hz); 7.42 (d, 1H, J = 8.7 Hz); 8.03 (s, 1H); 8.14 (s, 1H); 11.90 (br s, 1H); 12.64 (br s, 1H). | (-CI) *m*/*z* 469/470/ 471 | 3 |
| **15** | Diethyl-carbamic acid 5-(7-chloro-4-hydroxy-1,10-dioxo-5,10-dihydro-1*H*-pyridazino[4,5-*b*] quinolin-2-ylmethyl)-2-methoxy-phenyl ester. | 3% | 1.09 (m, 3H); 1.17 (m, 3H); 3.26 (m, 2H); 3,37 (m, 2H); 3.73 (s, 3H); 5.03 (s, 2H); 7.04 (m, 2H); 7.13 (m, 1H); 7.43 (d, 1H, J = 8.7 Hz); 8.03 (s, 1H); 8.14 (d, 1H, J = 8.7 Hz); 11.90 (br s, 1H); 12.63 (br s, 1H). | (-CI) *m*/*z* 497/498/ 499 | 3 |

Table 1: (continued)

| Ex. | Compound Name | Yield | NMR (300 MHz, DMSO-$d_6$): δ | MS | Method |
|---|---|---|---|---|---|
| **16** | Morpholine-4-carboxylic acid 5-(7-chloro-4-hydroxy-1,10-dioxo-5,10-dihydro-1*H*-pyridazino[4,5-*b*]quinolin-2-ylmethyl)-2-methoxy-phenyl ester. | 10% | 3.37 (m, 4H); 3.62 (m, 2H); 5.03 (s, 2H); 7.05 (m, 2H); 7.15 (m, 1H); 7.43 (d, 1H, J = 8.7 Hz); 8.02 (s, 1H); 8.14 (d, 1H, J = 8.7 Hz); 11.89 (br s, 1H); 12.61 (br s, 1H). | (-CI) *m/z* 511/512 | 3 |

**Example 17:** 7-Chloro-4-hydroxy-2-[4-ethoxycarbonyloyl-3,5-dimethoxybenzyl]-1,2,5,10-tetrahydropyridazino[4,5-*b*]quinoline-1,10-dione.

4-Ethoxycarbonyloyl-3,5-dimethoxybenzyl alcohol

To a solution of 4-ethoxycarbonyloyl-3,5-dimethoxybenzoic acid (2.1 g, 7.8 mmol) in THF (40 mL) was added borane dimethylsulfide (5 mL of a 10 M sol'n, 50 mmol). The mixture was stirred for 3 h. under nitrogen, at which time the volatiles were removed by rotary evaporation. The residue was taken up in ethyl acetate (100 mL) and washed twice with water (50 mL) then brine. The organic phase was dried with $MgSO_4$ and concentrated to a colorless oil by rotary evaporation. Upon standing overnight the material crystallized. This solid was triturated with hexanes to afford the desired material (1.82 g, 91 %) as a white solid of sufficient purity to proceed to the next reaction. $^1$H NMR (DMSO-$d_6$): δ 6.71 (s, 2H); 5.25 (s, 1H); 4.48 (s, 2H); 4.20 (q, *J* = 7.2 Hz, 2H); 3.76 (s, 6H); 1.26 (t, *J* = 7.2 Hz, 3H).

4-Ethoxycarbonyloyl-3,5-dimethoxybenzaldehyde

A solution of oxalyl chloride (1.31 mL, 15 mmol) in DCM (100 mL) was cooled to - 60 °C under a nitrogen atmosphere. A solution of DMSO (2.1 mL, 30 mmol) in DCM (8 mL) was added dropwise over 10 min while maintaining the reaction temperature below -50 °C. A solution of 4-ethoxycarbonyloyl-3,5-dimethylbenzyl alcohol (1.8 g, 7.0 mmol) in DCM (10 mL) was added. The mixture was allowed to react for 1 h at -70 °C. A solution of triethylamine (8 mL, 60 mmol) in DCM (10 mL) was added dropwise. The cooling bath was removed and the reaction was allowed to warm to room temperature. After 1 h the reaction was quenched with water (50 mL) and the phases were separated. The organic phase was washed with water (50 mL) three times, then brine and dried over $MgSO_4$. Rotary evaporation afforded a tan solid that was triturated with 1:1 diethyl ether:hexanes. The obtained solid was dried *in vacuo* for 30 min to afford the title compound (1.66 g, 93 %) as a tan solid. $^1$H NMR (300 MHz, DMSO-$d_6$): δ 9.96 (s, 1H); 7.35 (s, 2H); 4.25 (q, *J* = 7.2 Hz, 2H); 3.88 (s, 6H); 1.28 (t, *J* = 7.2 Hz, 3H).

7-Chloro-4-hydroxy-2-[4-ethoxycarbonyloyl-3,5-dimethoxybenzyl]-1,2,5,10-tetrahydropyridazino[4,5-*b*]quinoline-1,10-dione.

The title compound was prepared from 4-ethoxycarbonyloyl-3,5-dimethoxybenzaldehyde by the method of Example 2. $^1$H NMR (300 MHz, DMSO-$d_6$) δ 12.65 (s,1H); 11.92 (s, 1H): 8.15 (d, *J* = 8.7 Hz, 8.03 (s, 1H); 7.44 (d, *J* = 10.2 Hz, 1H); 6.70 (s, 2H); 5.09 (s, 2H); 4.20 (q, *J* = 7.2 Hz, 2H); 3.74 (s, 6H); 1.26 (t, *J* = 7.2 Hz, 3H). Calc'd. for $C_{23}H_{20}ClN_3O_8$: C, 55.04; H, 4.02; N, 8.37; Found: C, 54.82, 54.71; H, 4.01, 3.99; N,8.25, 8.16.

**Tests for Biological Function:**

**Test A:** Inhibition of binding of [$^3$H]-MDL105,519:

Binding of compounds to the NMDA receptor glycine site may be assessed by measuring the ability of test compounds to inhibit the binding of tritiated MDL105,519 to brain membranes bearing the receptor.

Rat Brain Membranes: The rat brain membranes used in the experiments were obtained from Analytical Biological Services Inc., and were prepared substantially in accordance with the method of B.M. Baron *et al*., *J. Pharmacol. Exp. Ther.* 250, 162 (1989). Briefly, fresh brain tissue including cerebral cortex and hippocampus from male Sprague Dawley rats was homogenized in 0.32 M sucrose and centrifuged at low speed to separate cellular membranes from other cellular components. The membranes were then washed 3 times using deionized water, followed by treatment with 0.04% Triton X-100. Finally, membranes were washed six times in 50 mM Tris citrate buffer, pH 7.4, and

frozen at -80 °C until use.

[$^3$H]MDL105,519 (72 Ci/mmol) was purchased from Amersham. Cold MDL105,519 was purchased from Sigma/RBI. Binding assays were performed substantially in accordance with the protocol of B.M. Baron *et al., J. Pharmacol. Exp. Ther.* 279, 62 (1996), as follows. On the day of the experiment, brain membranes were thawed at room temperature and suspended in 50 mM tris acetate buffer, pH 7.4 ("TAB"). Seventy-five micro grams per milliliter protein (by using the BioRad dye) were used for competition binding. The experiments were carried out using 96-well plates. Membranes were incubated with 20 μL of compounds of various concentrations and 1.2 nM [$^3$H]MDL105,519 for 30 minutes at room temperature in a total volume of 250 μL. Non specific binding was determined by using 100 μM of unlabeled MDL105,519. The unlabeled MDL105,519 and compounds were dissolved as 12.5 mM stock solutions in DMSO. Final DMSO concentration in each well was kept below 1%, which concentration was found not to alter the binding results. After incubation, unbound [$^3$H]MDL105,519 was removed by filtration onto GF/B Unifilter plates using a Packard harvester. Filters were washed four times with ice cold TAB (total of 1.2 mL buffer). The plates were dried overnight at room temperature and bound radioactivity was measured on a Packard TopCount after the addition of 45 μL per well of the MICROSCINT O.

Human Brain Membranes: Human brain membranes were obtained from Analytical Biological Services Inc., and assays were performed as described for rat membranes.

Data analysis: Data was analyzed using a Microsoft Excel spreadsheet and GraphPad Prizm software and potency of compounds is expressed as the Ki (nM).

**Test B:** Formalin test:

The Formalin test is an assay that assesses the capacity of a compound to inhibit formalin-induced nociceptive behaviors in rats (D. Dubuisson, *et al., Pain* 4, 161-174 (1977); H. Wheeler-Aceto *et al., Psychopharmacology* 104, 35-44 (1991); T.J. Coderre, *et al., Pain* 54, 43-50 (1993)). In the test, two distinctive phases of formalin-induced behaviors are observed. A first phase response, caused by acute nociception to the noxious chemical (formalin) injected into the paw, occurs between zero and five minutes. A quiescent period of 5 to 15 min post injection follows. After the quiescent period a second phase response, caused by sensitization of the central neurons in the dorsal horn, occurs after 15 minutes and lasts up to 60 minutes. Sensitization of the central neurons in the spine augments a noxious afferent input and causes a stronger pain barrage to be transmitted to the brain. Therefore, inhibition of the second phase response indicates a central mechanism of drug action.

The procedure for the formalin test may be performed as follows: male rats are placed in a plexiglass chamber and observed for 30-45 min. to observe their baseline activity. Animals would either be pretreated with vehicle or with different doses of a test compound and are dosed with vehicle or test compound three hours prior to injection of 0.05 mL of sterile 1% formalin under the dorsal skin of a hind paw. The number of paw flinches (responses) during the first phase (0-5 min.) and the second phase (20-35 min.) are scored and recorded. Flinch response can be compared with the mean score of a saline control group and calculated as percentage inhibition. The $ED_{50}$ may be determined form the dose of compound which produces 50% inhibition of nociceptive response in the first or second phase response. First phase responses may be inhibited by compounds that act peripherally and by compounds that act centrally. Second phase response are inhibited by centrally active compounds.

$$\text{\% inhibition of nociceptive response} = 100 \times \frac{\text{(number of responses in vehicle group - number of responses in compound group)}}{\text{(number of responses in vehicle group)}}$$

Student's t-test may be used for statistical analysis to determine the significance of compound effects.

**Test C:** Neuropathic pain model (Chronic Constriction Injury):

The anti-hyperalgesic properties of a compound may be tested with the Chronic Constriction Injury ("CCI") model. The test is a model for neuropathic pain associated with nerve injuries that can arise directly from trauma and compression, or indirectly from a wide range of diseases such as infection, cancer, metabolic conditions, toxins, nutritional deficiencies, immunological dysfunction, and musculoskeletal changes. In the model a unilateral peripheral hyperalgesia is produced in rats by nerve ligation (G.J. Bennett, *et al.*, *Pain* 33, 87-107 (1988)).

Procedurally, Sprague-Dawley rats (250-350 g) are anesthetized with sodium pentobarbital and the common sciatic nerve is exposed at the level of the mid thigh by blunt dissection through the biceps femoris. A section of nerve (about 7 mm), proximal to the sciatic trifucation, is freed of tissue and ligated at four positions with chromic gut suture. The suture is tied with about 1 mm spacing between ligatures. The incision is closed in layers and the animals are

allowed to recuperate. Thermal hyperalgesia is measured using a paw-withdrawal test (K. Hargreaves, *et al., Pain* 32, 77-88 (1988)). To perform the test, animals are habituated on an elevated glass floor. A radiant heat source is aimed at the mid-plantar hindpaw (sciatic nerve territory) through the glass floor with a 20 second cut-off used to prevent injury to the skin. The latencies for the withdrawal reflex in both hind paws are recorded.

Injured paws with ligated nerves show shorter paw withdrawal latencies compared to the uninjured or sham operated paws. Responses to test compounds are evaluated at different times after oral administration to determine the onset and duration of compound effect. When performing the test, groups of CCI rats receive either vehicle or the test compound orally three times daily for 5 days. Paw withdrawal latencies are measured each day 10 min before and 2 or 3 hr. after the first daily dose. Compound efficacy is expressed as mean percentage decrease of hyperalgesia compared to that of vehicle-treated animals, calculated as follows:

$$\frac{\text{(Mean of vehicle group - Mean of compound group)}}{\text{(Mean of vehicle group).}} \times 100$$

Data analysis was performed by the multiple means comparison test (Dunnett's test) and results are expressed and compound potencies are expressed as the MED (minimum effective dose), in mg/Kg/day, that yields a percent (%) decrease in hyperalgesia that is statistically significant.

Table 2 shows the results from Tests A and C for certain compounds of the invention. Where no data is provided in the table, the test was not performed.

Table 2

| **Example No.** | **Test A Ki (nM)** | **Test C MED (%Inb.)** |
|---|---|---|
| Ex. 1 | 267 | |
| Ex. 2 | 59 | |
| Ex. 3 | 165 | |
| Ex. 4 | 146 | |
| Ex. 5 | 86.6 | |
| Ex. 6 | 75.9 | |
| Ex. 7 | 106 | 15 (15%) |
| Ex. 8 | 166 | |
| Ex. 9 | 83.4 | |
| Ex. 10 | 248 | |
| Ex. 11 | 57.4 | |
| Ex. 12 | 158 | |
| Ex. 13 | 205 | |
| Ex. 14 | 37.3 | |
| Ex. 15 | 27.9 | |
| Ex. 16 | 31.3 | |
| Ex. 17 | 79 | |

## Claims

**1.** The compounds selected from;
7-chloro-4-hydroxy-2-(4-dimethylcarbamoylbenzyl)-1,2,5,10-tetrahydropyridazino[4,5-*b*]quinoline-1,10-dione,
7-chloro-4-hydroxy-2-[3-methyl-4-(pyridin-2-ylmethoxy)-benzyl]-1,2,5,10-tetrahydropyridazino[4,5-*b*]quinoline-1,10-dione,
7-chloro-4-hydroxy-2-(4-dimethylcarbamoyl(3-methyl)benzyl)-1,2,5,10-tetrahydropyridazino[4,5-*b*]quinoline-1,10-dione,

dimethyl-carbamic acid 3-(7-chloro-4-hydroxy-1,10-dioxo-5,10-dihydro-1*H*-pyridazino[4,5-*b*]quinolin-2-ylmethyl)-phenyl ester,
methyl-phenyl-carbamic acid 4-(7-chloro-4-hydroxy-1,10-dioxo-5,10-dihydro-1*H* -pyridazino[4,5-*b*]quinolin-2-ylmethyl)-phenyl ester,
diethyl-carbamic acid 4-(7-chloro-4-hydroxy-1,10-dioxo-5,10-dihydro-1*H*-pyridazino[4,5-*b*]quinolin-2-ylmethyl)-phenyl ester,
diethyl-carbamic acid 3-(7-chloro-4-hydroxy-1,10-dioxo-5,10-dihydro-1*H*-pyridazino[4,5-*b*]quinolin-2-ylmethyl)-phenyl ester,
morpholine-4-carboxylic acid 3-(7-chloro-4-hydroxy 1,10-dioxo-5,10-dihydro-1*H* -pyridazino[4,5-*b*]quinolin-2-ylmethyl)-phenyl ester,
methyl-phenyl-carbamic acid 3-(7-chloro-4-hydroxy-1,10-dioxo-5,10-dihydro-1*H* -pyridazino[4,5-*b*]quinolin-2-ylmethyl)-phenyl ester,
diphenyl-carbamic acid 3-(7-chloro-4-hydroxy-1,10-dioxo-5,10-dihydro-1*H*-pyridazino[4,5-*b*]quinolin-2-ylmethyl)-phenyl ester,
pyrrolidine-1-carboxylic acid 3-(7-chloro-4-hydroxy-1,10-dioxo-5,10-dihydro-1*H* -pyridazino[4,5-*b*]quinolin-2-ylmethyl)-phenyl ester,
morpholine-4-carboxylic acid 4-(7-chloro-4-hydroxy-1,10-dioxo-5,10-dihydro-1*H* -pyridazino[4,5-*b*]quinolin-2-ylmethyl)-2-methyl-phenyl ester,
methyl-phenyl-carbamic acid 4-(7-chloro-4-hydroxy-1,10-dioxo-5,10-dihydro-1*H* -pyridazino[4,5-*b*]quinolin-2-ylmethyl)-2-methyl-phenyl ester,
dimethyl-carbamic acid 5-(7-chloro-4-hydroxy-1,10-dioxo-5,10-dihydro-1*H*-pyridazino[4,5-*b*]quinolin-2-ylmethyl)-2-methoxy-phenyl ester,
diethyl-carbamic acid 5-(7-chloro-4-hydroxy-1,10-dioxo-5,10-dihydro-1*H*-pyridazino[4,5-*b*]quinolin-2-ylmethyl)-2-methoxy-phenyl ester,
morpholine-4-carboxylic acid 5-(7-chloro-4-hydroxy-1,10-dioxo-5,10-dihydro-1*H* -pyridazino[4,5-*b*]quinolin-2-ylmethyl)-2-methoxy-phenyl ester, and
7-chloro-4-hydroxy-2-[4-ethoxycarbonyloyl-3,5-dimethoxybenzyl]-1,2,5,10-tetrahydropyridazino[4,5-*b*]quinoline-1,10-dione,
and pharmaceutically-acceptable salts thereof.

**2.** Use of a compound selected from;
7-chloro-4-hydroxy-2-(4-dimethylcarbamoylbenzyl)-1,2,5,10-tetrahydropyridazino[4,5-*b*]quinoline-1,10-dione,
7-chloro-4-hydroxy-2-[3-methyl-4-(pyridin-2-ylmethoxyrbenzyl]-1,2,5,10-tetrahydropyridazino[4,5-*b*]quinoline-1,10-dione,
7-chloro-4-hydroxy-2-(4-dimethylcarbamoyl(3-methyl)benzyl)-1,2,5,10-tetrahydropyridazino[4,5-*b*]quinoline-1,10-dione,
dimethyl-carbamic acid 3-(7-chloro-4-hydroxy-1,10-dioxo-5,10-dihydro-1*H*-pyridazino[4,5-*b*]quinolin-2-ylmethyl)-phenyl ester,
methyl-phenyl-carbamic acid 4-(7-chloro-4-hydroxy-1,10-dioxo-5,10-dihydro-1*H* -pyridazino[4,5-*b*]quinolin-2-ylmethyl)-phenyl ester,
diethyl-carbamic acid 4-(7-chloro-4-hydroxy-1,10-dioxo-5,10-dihydro-1*H*-pyridazino[4,5-*b*]quinolin-2-ylmethyl)-phenyl ester,
diethyl-carbamic acid 3-(7-chloro-4-hydroxy-1,10-dioxo-5,10-dihydro-1*H*-pyridazino[4,5-*b*]quinolin-2-ylmethyl)-phenyl ester,
morpholine-4-carboxylic acid 3-(7-chloro-4-hydroxy-1,10-dioxo-5,10-dihydro-1*H* -pyridazino[4,5-*b*]quinolin-2-ylmethyl)-phenyl ester,
methyl-phenyl-carbamic acid 3-(7-chloro-4-hydroxy-1,10-dioxo-5,10-dihydro-1*H* -pyridazino[4,5-*b*]quinolin-2-ylmethyl)-phenyl ester,
diphenyl-carbamic acid 3-(7-chloro-4-hydroxy-1,10-dioxo-5,10-dihydro-1*H*-pyridazino[4,5-*b*]quinolin-2-ylmethyl)-phenyl ester,
pyrrolidine-1-carboxylic acid 3-(7-chloro-4-hydroxy-1,10-dioxo-5,10-dihydro-1*H* -pyridazino[4,5-*b*]quinolin-2-ylmethyl)-phenyl ester,
morpholine-4-carboxylic acid 4-(7-chloro-4-hydroxy-1,10-dioxo-5,10-dihydro-1*H* -pyridazino[4,5-*b*]quinolin-2-ylmethyl)-2-methyl-phenyl ester,
methyl-phenyl-carbamic acid 4-(7-chloro-4-hydroxy-1,10-dioxo-5,10-dihydro-1*H* -pyridazino[4,5-*b*]quinolin-2-ylmethyl)-2-methyl-phenyl ester,
dimethyl-carbamic acid 5-(7-chloro-4-hydroxy-1,10-dioxo-5,10-dihydro-1*H*-pyridazino[4,5-*b*]quinolin-2-ylmethyl)-2-methoxy-phenyl ester,

diethyl-carbamic acid 5-(7-chloro-4-hydroxy-1,10-dioxo-5,10-dihydro-1*H*-pyridazino[4,5-*b*]quinolin-2-ylmethyl)-2-methoxy-phenyl ester,
morpholine-4-carboxylic acid 5-(7-chloro-4-hydroxy-1,10-dioxo-5,10-dihydro-1*H* -pyridazino[4,5-*b*]quinolin-2-ylmethyl)-2-methoxy-phenyl ester, and
7-chloro-4-hydroxy-2-[4-ethoxycarbonyloyl-3,5-dimethoxybenzyl]-1,2,5,10-tetrahydropyridazino[4,5-*b*]quinoline-1,10-dione,
and pharmaceutically-acceptable salts thereof, in the manufacture of a medicament for the treatment of neuropathic pain.

**3.** A pharmaceutical compositions comprising a pain-ameliorating effective amount of a compound according to claim 1 in association with a pharmaceutically-acceptable excipient or diluent

**Patentansprüche**

**1.** Verbindungen ausgewählt aus:

7-Chlor-4-hydroxy-2-(4-dimethylcarbamoylbenzyl)-1,2,5,10-tetrahydropyridazino[4,5-*b*]chinolin-1,10-dion,
7-Chlor-4-hydroxy-2-[3-methyl-4-(pyridin-2-ylmethoxy)benzyl]-1,2,5,10-tetrahydropyridazino[4,5-*b*]chinolin-1,10-dion,
7-Chlor-4-hydroxy-2-(4-dimethylcarbamoyl-(3-methyl)benzyl)-1,2,5,10-tetrahydropyridazino[4,5-*b*]chinolin-1,10-dion,
Dimethylcarbaminsäure-3-(7-chlor-4-hydroxy-1,10-dioxo-5,10-dihydro-1H-pyridazino[4,5-*b*]chinolin-2-ylmethyl)phenylester,
Methylphenylcarbaminsäure-4-(7-chlor-4-hydroxy-1,10-dioxo-5,10-dihydro-1H-pyridazino[4,5-*b*]chinolin-2-ylmethyl)phenylester,
Diethylcarbaminsäure-4-(7-chlor-4-hydroxy-1,10-dioxo-5,10-dihydro-1H-pyridazino[4,5-*b*]chinolin-2-ylmethyl)phenylester,
Diethylcarbaminsäure-3-(7-chlor-4-hydroxy-1,10-dioxo-5,10-dihydro-1H-pyridazino[4,5-*b*]chinolin-2-ylmethyl)phenylester,
Morpholin-4-carbonsäure-3-(7-chlor-4-hydroxy-1,10-dioxo-5,10-dihydro-1H-pyridazino[4,5-*b*]chinolin-2-ylmethyl)phenylester,
Methylphenylcarbaminsäure-3-(7-chlor-4-hydroxy-1,10-dioxo-5,10-dihydro-1H-pyridazino[4,5-*b*]chinolin-2-ylmethyl)phenylester,
Diphenylcarbaminsäure-3-(7-chlor-4-hydroxy-1,10-dioxo-5,10-dihydro-1H-pyridazino[4,5-*b*]chinolin-2-ylmethyl)phenylester,
Pyrrolidin-1-carbonsäure-3-(7-chlor-4-hydroxy-1,10-dioxo-5,10-dihydro-1H-pyridazino[4,5-*b*]chinolin-2-ylmethyl)phenylester,
Morpholin-4-carbonsäure-4-(7-chlor-4-hydroxy-1,10-dioxo-5,10-dihydro-1H-pyridazino[4,5-*b*]chinolin-2-ylmethyl)-2-methylphenylester,
Methylphenylcarbaminsäure-4-(7-chlor-4-hydroxy-1,10-dioxo-5,10-dihydro-1H-pyridazino[4,5-*b*]chinolin-2-ylmethyl)-2-methylphenylester,
Dimethylcarbaminsäure-5-(7-chlor-4-hydroxy-1,10-dioxo-5,10-dihydro-1H-pyridazino[4,5-*b*]chinolin-2-ylmethyl)-2-methoxyphenylester,
Diethylcarbaminsäure-5-(7-chlor-4-hydroxy-1,10-dioxo-5,10-dihydro-1H-pyridazino[4,5-*b*]chinolin-2-ylmethyl)-2-methoxyphenylester,
Morpholin-4-carbonsäure-5-(7-chlor-4-hydroxy-1,10-dioxo-5,10-dihydro-1H-pyridazino[4,5-*b*]chinolin-2-ylmethyl)-2-methoxyphenylester, und
7-Chlor-4-hydroxy-2-[4-ethoxycarbonyloyl-3,5-dimethoxybenzyl]-1,2,5,10-tetrahydropyridazino-[4,5-*b*]chinolin-1,10-dion,

und deren pharmazeutisch unbedenklichen Salzen.

**2.** Verwendung einer Verbindung ausgewählt aus:

7-Chlor-4-hydroxy-2-(4-dimethylcarbamoylbenzyl)-1,2,5,10-tetrahydropyridazino[4,5-*b*]chinolin-1,10-dion,
7-Chlor-4-hydroxy-2-[3-methyl-4-(pyridin-2-ylmethoxy)benzyl]-1,2,5,10-tetrahydropyridazino[4,5-*b*]chinolin-1,10-dion,

7-Chlor-4-hydroxy-2-(4-dimethylcarbamoyl-(3-methyl)benzyl)-1,2,5,10-tetrahydropyridazino[4,5-*b*]chinolin-1,10-dion,
Dimethylcarbaminsäure-3-(7-chlor-4-hydroxy-1,10-dioxo-5,10-dihydro-1H-pyridazino[4,5-*b*]chinolin-2-ylmethyl)phenylester,
Methylphenylcarbaminsäure-4-(7-chlor-4-hydroxy-1,10-dioxo-5,10-dihydro-1H-pyridazino[4,5-*b*]chinolin-2-ylmethyl)phenylester,
Diethylcarbaminsäure-4-(7-chlor-4-hydroxy-1,10-dioxo-5,10-dihydro-1H-pyridazino[4,5-*b*]chinolin-2-ylmethyl)phenylester,
Diethylcarbaminsäure-3-(7-chlor-4-hydroxy-1,10-dioxo-5,10-dihydro-1H-pyridazino[4,5-*b*]chinolin-2-ylmethyl)phenylester,
Morpholin-4-carbonsäure-3-(7-chlor-4-hydroxy-1,10-dioxo-5,10-dihydro-1H-pyridazino[4,5-*b*]chinolin-2-ylmethyl)phenylester,
Methylphenylcarbaminsäure-3-(7-chlor-4-hydroxy-1,10-dioxo-5,10-dihydro-1H-pyridazino[4,5-*b*]chinolin-2-ylmethyl)phenylester,
Diphenylcarbaminsäure-3-(7-chlor-4-hydroxy-1,10-dioxo-5,10-dihydro-1H-pyridazino[4,5-*b*]chinolin-2-ylmethyl)phenylester,
Pyrrolidin-1-carbonsäure-3-(7-chlor-4-hydroxy-1,10-dioxo-5,10-dihydro-1H-pyridazino[4,5-*b*]chinolin-2-ylmethyl)phenylester,
Morpholin-4-carbonsäure-4-(7-chlor-4-hydroxy-1,10-dioxo-5,10-dihydro-1H-pyridazino[4,5-*b*]chinolin-2-ylmethyl)-2-methylphenylester,
Methylphenylcarbaminsäure-4-(7-chlor-4-hydroxy-1,10-dioxo-5,10-dihydro-1H-pyridazino[4,5-*b*]chinolin-2-ylmethyl)-2-methylphenylester,
Dimethylcarbaminsäure-5-(7-chlor-4-hydroxy-1,10-dioxo-5,10-dihydro-1H-pyridazino[4,5-*b*]chinolin-2-ylmethyl)-2-methoxyphenylester,
Diethylcarbaminsäure-5-(7-chlor-4-hydroxy-1,10-dioxo-5,10-dihydro-1H-pyridazino[4,5-*b*]chinolin-2-ylmethyl)-2-methoxyphenylester,
Morpholin-4-carbonsäure-5-(7-chlor-4-hydroxy-1,10-dioxo-5,10-dihydro-1H-pyridazino[4,5-*b*]chinolin-2-ylmethyl)-2-methoxyphenylester, und
7-Chlor-4-hydroxy-2-[4-ethoxycarbonyloyl-3,5-dimethoxybenzyl]-1,2,5,10-tetrahydropyridazino-[4,5-*b*]chinolin-1,10-dion,

und deren pharmazeutisch unbedenklichen Salzen bei der Herstellung eines Medikaments zur Behandlung neuropathischer Schmerzen.

**3.** Pharmazeutische Zusammensetzungen, enthaltend eine schmerzlindernde Menge einer Verbindung nach Anspruch 1 zusammen mit einem pharmazeutisch unbedenklichen Träger oder Verdünnungsmittel.

**Revendications**

**1.** Composés choisis parmi :

la 7-chloro-4-hydroxy-2-(4-diméthylcarbamoylbenzyl)-1,2,5,10-tétrahydropyridazino[4,5-*b*]-quinoléine-1,10-dione,
la 7-chloro-4-hydroxy-2-[3-méthyl-4-(pyridin-2-yl-méthoxy)benzyl]-1,2,5,10-tétrahydropyridazino[4,5-*b*]quinoléine-1,10-dione,
la 7-chloro-4-hydroxy-2-(4-diméthylcarbamoyl(3-méthyl)bênzyl)-1,2,5,10-tétrahydropyridazino[4,5-*b*]quinoléine-1,10-dione,
l'ester 3-(7-chloro-4-hydroxy-1,10-dioxo-5,10-dihydro-1H-pyridazino[4,5-*b*]quinoléin-2-ylméthyl)phénylique d'acide diméthylcarbamique,
l'ester 4-(7-chloro-4-hydroxy-1,10-dioxo-5,10-dihydro-1H-pyridazino[4,5-*b*]quinoléin-2-ylméthyl)-phénylique d'acide méthylphénylcarbamique,
l'ester 4-(7-chloro-4-hydroxy-1,10-dioxo-5,10-dihydro-1H-pyridazino[4,5-*b*]quinoléin-2-ylméthyl)phénylique d'acide diéthylcarbamique,
l'ester 3-(7-chloro-4-hydroxy-1,10-dioxo-5,10-dihydro-1H-pyridazino[4,5-*b*]quinoléin-2-ylméthyl)phénylique d'acide diéthylcarbamique,
l'ester 3-(7-chloro-4-hydroxy-1,10-dioxo-5,10-dihydro-1H-pyridazino[4,5-*b*]quinoléin-2-ylméthyl)phénylique d'acide morpholine-4-carboxylique,

l'ester 3-(7-chloro-4-hydroxy-1,10-dioxo-5,10-dihydro-1H-pyridazino[4,5-*b*]quinoléin-2-ylméthyl)-phénylique d'acide méthylphénylcarbamique,
l'ester 3-(7-chloro-4-hydroxy-1,10-dioxo-5,10-dihydro-1H-pyridazino[4,5-*b*]quinoléin-2-ylméthyl)phénylique d'acide diphénylcarbamique,
l'ester 3-(7-chloro-4-hydroxy-1,10-dioxo-5,10-dihydro-1H-pyridazino[4,5-*b*]quinoléin-2-ylméthyl)phénylique d'acide pyrrolidine-1-carboxylique,
l'ester 4-(7-chloro-4-hydroxy-1,10-dioxo-5,10-dihydro-1H-pyridazino[4,5-*b*]quinoléin-2-ylméthyl)-2-méthylphénylique d'acide morpholine-4-carboxylique,
l'ester 4-(7-chloro-4-hydroxy-1,10-dioxo-5,10-dihydro-1H-pyridazino[4,5-*b*]quinoléin-2-ylméthyl)-2-méthylphénylique d'acide méthylphénylcarbamique,
l'ester 5-(7-chloro-4-hydroxy-1,10-dioxo-5,10-dihydro-1H-pyridazino[4,5-*b*]quinoléin-2-ylméthyl)-2-méthoxyphénylique d'acide diméthylcarbamique,
l'ester 5-(7-chloro-4-hydroxy-1,10-dioxo-5,10-dihydro-1H-pyridazino[4,5-*b*)quinoléin-2-ylméthyl)-2-méthoxyphénylique d'acide diéthylcarbamique,
l'ester 5-(7-chloro-4-hydroxy-1,10-dioxo-5,10-dihydro-1H-pyridazino[4,5-*b*]quinoléin-2-ylméthyl)-2-méthoxyphénylique d'acide morpholine-4-carboxylique, et
la 7-chloro-4-hydroxy-2-[4-éthoxycarbonyloyl-3,5-diméthoxybenzyl]-1,2,5,10-tétrahydropyridazino-[4,5-*b*]quinoléine-1,10-dione,

et sels pharmaceutiquement acceptables de ces composés.

**2.** Utilisation d'un composé choisi parmi :

la 7-chloro-4-hydroxy-2-(4-diméthylcarbamoylbenzyl)-1,2,5,10-tétrahydropyridazino[4,5-*b*]quinoléine-1,10-dione,
la 7-chloro-4-hydroxy-2-[3-méthyl-4-(pyridin-2-yl-méthoxy)benzyl]-1,2,5,10-tétrahydropyridazino[4,5-*b*]quinoléine-1,10-dione,
la 7-chloro-4-hydroxy-2-(4-diméthylcarbamoyl(3-méthyl)benzyl)-1,2,5,10-tétrahydropyridazino[4,5-*b*]quinoléine-1,10-dione,
l'ester 3-(7-chloro-4-hydroxy-1,10-dioxo-5,10-dihydro-1H-pyridazino[4,5-*b*]quinoléin-2-ylméthyl)phénylique d'acide diméthylcarbamique,
l'ester 4-(7-chloro-4-hydroxy-1,10-dioxo-5,10-dihydro-1H-pyridazino[4,5-b]quinoléin-2-ylméthyl)phénylique d'acide méthylphénylcarbamique,
l'ester 4-(7-chloro-4-hydroxy-1,10-dioxo-5,10-dihydro-1H-pyridazino[4,5-b]quinoléin-2-ylméthyl)phénylique d'acide diéthylcarbamique,
l'ester 3-(7-chloro-4-hydroxy-1,10-dioxo-5,10-dihydro-1H-pyridazino[4,5-*b*]quinoléin-2-ylméthyl)phénylique d'acide diéthylcarbamique,
l'ester 3-(7-chloro-4-hydroxy-1,10-dioxo-5,10-dihydro-1H-pyridazino[4,5-*b*]quinoléin-2-ylméthyl)phénylique d'acide morpholine-4-carboxylique,
l'ester 3-(7-chloro-4-hydroxy-1,10-dioxo-5,10-dihydro-1H-pyridazino[4,5-*b*]quinoléin-2-ylméthyl)phénylique d'acide méthylphénylcarbamique,
l'ester 3-(7-chloro-4-hydroxy-1,10-dioxo-5,10-dihydro-1H-pyridazino[4,5-*b*]quinoléin-2-ylméthyl)phénylique d'acide diphénylcarbamique,
l'ester 3-(7-chloro-4-hydroxy-1,10-dioxo-5,10-dihydro-1H-pyridazino[4,5-*b*]quinoléin-2-ylméthyl)phénylique d'acide pyrrolidine-1-carboxylique,
l'ester 4-(7-chloro-4-hydroxy-1,10-dioxo-5,10-dihydro-1H-pyridazino[4,5-*b*]quinoléin-2-ylméthyl)-2-méthylphénylique d'acide morpholine-4-carboxylique,
l'ester 4-(7-chloro-4-hydroxy-1,10-dioxo-5,10-dihydro-1H-pyridazino[4,5-*b*]quinoléin-2-ylméthyl)-2-méthylphénylique d'acide méthylphénylcarbamique,
l'ester 5-(7-chloro-4-hydroxy-1,10-dioxo-5,10-dihydro-1H-pyridazino[4,5-*b*]quinoléin-2-ylméthyl)-2-méthoxyphénylique d'acide diméthylcarbamique,
l'ester 5-(7-chloro-4-hydroxy-1,10-dioxo-5,10-dihydro-1H-pyridazino[4,5-*b*]quinoléin-2-ylméthyl)-2-méthoxyphénylique d'acide diéthylcarbamique,
l'ester 5-(7-chloro-4-hydroxy-1,10-dioxo-5,10-dihydro-1H-pyridazino[4,5-*b*]quinoléin-2-ylméthyl)-2-méthoxyphénylique d'acide morpholine-4-carboxylique, et
la 7-chloro-4-hydroxy-2-[4-éthoxycarbonyloyl-3,5-diméthoxybenzyl]-1,2,5,10-tétrahydropyridazino[4,5-*b*]quinoléine-1,10-dione,

et de sels pharmaceutiquement acceptables de ces composés, dans la fabrication d'un médicament destiné au traitement d'une douleur névropathique.

3. Compositions pharmaceutiques comprenant une quantité efficace pour améliorer la douleur d'un composé selon la revendication 1 en association avec un excipient ou diluant pharmaceutiquement acceptable.